# EUROPEAN PATENT APPLICATION

(11) **EP 2 047 852 A1**
(43) Date of publication of application: **15.04.2009**
(21) Application number: 07788661.2
(22) Date of filing: 11.07.2007
(51) Int. Cl.: A61K 31/5517, A61P 25/24

(54) **USE OF FLUMAZENIL IN THE PRODUCTION OF A MEDICAMENT FOR THE TREATMENT OF DEPRESSIVE DISORDERS**

(30) Priority: 14.07.2006 ES 200601893
(71) Applicant: Legarda Ibáñez, Juan José, 28109 Alcobendas, Madrid (ES)
(72) Inventor: Legarda Ibáñez, Juan José, 28109 Alcobendas, Madrid (ES)
(74) Representative: Maldonado Jordan, Julia
(86) International application number: PCT/ES2007/000415
(87) International publication number: WO 2008/006918

(57) **Abstract**

The invention relates to the use of flumazenil in the manufacture of a medicament for the treatment of depressive disorders, for example disorders including depressive symptoms, without psychotic alteration. The flumazenil can be administered sequentially in small quantities at short intervals until a quantity that is therapeutically effective for the treatment of the depressive disorder has been administered.

## Description

### Field of the Invention

The invention relates to the use of pharmaceutical compositions containing flumazenil in the treatment of depressive disorders, particularly in the treatment of disorders including depressive symptoms, without psychotic alteration.

### Background of the Invention

Depressive disorders are mental diseases including psychopathological disorders presented with mood or affective alterations in the sense of depression. This change is usually accompanied by a change in the general level of activity. Most of the remaining symptoms are secondary to these alterations or are comprehensive in their context. These disorders generally tend to be recurrent (i.e., recurrent depressive episodes occur) and the onset of each depressive episode is usually related to stressful situations or events.

In depressive episodes, the person who suffers them experiences a depressive mood, a loss of the capacity of being interested in and enjoying things, a reduced vitality, which leads to a reduced level of activity and exaggerated fatigue occurring even after a minimal effort. The following are also manifestations of depressive episodes: reduced attention and concentration; loss of self-esteem and feelings of inferiority; ideas of guilt and of being useless; a somber outlook of the future; suicidal or self-injuring thoughts and acts; sleep disorders; and loss of appetite.

A depressed mood varies little from one day to the next and does not usually respond to environmental changes, although it may show typical circadian variations. The clinical presentation may be different in each episode and in each individual. Atypical forms are particularly frequent during adolescence. In some cases, anxiety, unease and psychomotor agitation may predominate over the depression. The altered mood may be hidden by other symptoms, such as irritability, excessive alcohol consumption, histrionic behavior, exacerbation of phobias or pre-existing obsessive symptoms or by hypochondriacal preoccupations.

Some of the previous symptoms may be emphasized and acquire a special clinical meaning. The most typical examples of these "somatic" symptoms are: loss of interest or of the capacity to enjoy activities which were previously considered pleasant; loss of emotional reactivity to pleasant surrounding circumstances and events; waking up two or more hours than usual in the morning; deterioration of the depressive mood during the morning; objective presence of clear psychomotor inhibition or agitation (observed or reported by third persons); marked loss of appetite; weight loss (in the order of 5% or more of the body weight from the past month); and/or marked loss of sex drive. In general, if a patient presents four or more of said characteristics, he or she is considered to present "somatic" symptoms.

Depressive episodes can generally be classified into three categories (mild, moderate or severe). The mild, moderate and severe depressive episode categories are generally only used for isolated depressive episodes (or for the first episode). The following possible depressive episodes must be classified within one of the subdivisions of the recurrent depressive disorder. In general medical practice, patients with mild depressive episodes are frequent, whereas psychiatric wards usually deal with the more severe forms of depressive episodes.

A duration of at least two weeks is normally required for the diagnosis of a depressive episode of any of the three levels of severity (mild, moderate or severe), although shorter periods can be accepted if the symptoms are exceptionally severe or of a sudden onset.

The difference between the degrees of mild, moderate and severe is based on a complicated clinical assessment which includes the number, the type and the severity of the symptoms that are present. The level of the daily social and professional activity is usually a very useful general guideline for the severity of the episode, although personal, social and cultural factors having an influence on the relationship between the severity of the symptoms and social activity are frequent and intense enough so as to make it imprudent to include social functioning among the essential severity guidelines.

The International Classification of Diseases- Tenth Revision (ICD10), published by the World Health Organization (WHO), includes among depressive disorders, different disorders including depressive symptoms or episodes, such as those included within mood disorders (e.g., bipolar disorder depressive episodes, recurrent depressive disorders, persistent mood (affective) disorders, recurrent brief depressive disorder, etc.) and some neurotic, stress-related and somatoform disorders, such as mixed anxiety and depressive disorder, brief depressive reaction, prolonged depressive reaction and mixed anxiety and depressive reaction.

The treatments normally used to treat depressive disorders include somatic treatments, including pharmacological therapy and electroconvulsive therapy (when a patient does not respond to or does not tolerate pharmacotherapy, or when the clinical situation is so severe that a fast improvement is needed).

Included among the drugs normally used for the treatment of depressive disorders are cyclic antidepressants, for example, amitriptyline, amoxapine, bupropion, citalopram, clomipramine, desipramine, doxepin, imipramine, maprotiline, nefazodone, nortriptyline, protryptiline, trazodone, trimipramine, venlafaxine, etc., selective serotonin reuptake inhibitors (SSRIs), for example, fluoxetine, fluvoxamine, paroxetine, sertraline, etc., monoamine oxidase inhibitors (MAOIs), for example, isocarboxazid, pargyline, phenelzine, tranylcypromine, etc. Treatment with antidepressants generally lasts for at least 6 months, whereas a prophylactic intervention entails a consumption lasting for 5 years or more. A review of the different treatments of depressive disorders can be found in "Synopsis of Psychiatry", by Kaplan & Sadock, 7th edition, (1995), edited by Williams & Wilkins, Chapter 15.

The most widely used drugs are generally the SSRIs, although new drugs, such as bupropion, venlafaxine and nefazodone, are gaining acceptance. These drugs are safer than tricyclic antidepressants, tetracyclic antidepressants and MAOIs and on the other hand are just as effective. Tricyclic and tetracyclic antidepressants such as trazodone, alprazolam and mirtazapine, can cause sedation, while MAOIs require diet restrictions. One of the adverse effects of antidepressants which causes the most concern is their lethality when taken in high doses or mixed with alcohol. Other effects causing concern are those which act on the cardiovascular system and on sexuality causing a reduced sex drive, erectile dysfunction or anorgasmia as well as interactions with other drugs.

Although depressive disorders are relatively common mental diseases, treatment for a considerable number of patients is not completely satisfactory. For many years it has been considered that alterations in the monoamine function of the brain were underlying causes of affective disorders such as depression. Today it is known that the GABAergic system plays an important role in affective disorders. In fact, recent discoveries indicate that different anticonvulsants and GABA-mimetic drugs have antidepressant and mood-stabilizing properties. Different techniques [e.g., positron emission tomography (PET), single-photon emission computerized tomography (SPECT), magnetic resonance spectroscopy (MRS)] are providing evidence that GABAergic anomalies are associated with affective disorders such as depression. It has furthermore been found that depressed patients have lower gamma-aminobutyric acid (GABA) concentrations in the cerebrospinal fluid (CSF) and in plasma than non-depressed individuals.

Despite the fact that there are a number of antidepressants, there continue to be problems in the pharmacological treatment of depressive disorders. There are patients who do not respond to the first treatment, which must include maximum doses for a minimum period of 8 weeks because the antidepressants used take from 3 to 4 weeks to exert a significant therapeutic effect even though they may show an effect before that time. Another problem is based on the fact that the side effects of antidepressants require teaching patients how to tolerate symptoms such as agitation or gastrointestinal disorders. In addition, when prescribing these drugs it is important to take into account the quantities that are being prescribed given that most antidepressants are lethal if they are taken in large quantities. The use of some of the drugs used in the treatment of depressive disorders may likewise cause dependency and abstinence symptoms in addition to the aforementioned alterations.

Due to the fact that the drugs normally used for the treatment of depressive disorders cause a number of side effects, it is still necessary to find alternative drugs for the treatment of depressive disorders which overcome the aforementioned drawbacks and which at the same time are safe and effective.

Now it has surprisingly been found that flumazenil can be used safely and effectively in the treatment of depressive disorders.

Flumazenil [8-fluoro-5,6-dihydro-5-methyl-6-oxo-4H-imidazol[1,5-α][1,4]benzodiazepine-3-carboxylic acid ethyl ester] is a benzodiazepine antagonist which selectively blocks the effects exerted on the central nervous system through benzodiazepine receptors. This active ingredient is indicated for neutralizing the central sedating effect of benzodiazepines, therefore it is normally used in anesthesia to end general anesthesia induced and maintained with benzodiazepines in hospitalized patients, or to stop the sedation caused by benzodiazepines in patients subjected to short diagnostic or therapeutic procedures in a hospital or outpatient regimen.

The use of the flumazenil in the treatment of alcohol abstinence syndrome (WO 02/056964) and in the treatment of cocaine dependency (WO 02/064213) has also been described.

### Summary of the Invention

The object of the invention is based on providing a drug effective in the treatment of depressive disorders, particularly in the treatment of disorders including depressive symptoms, without psychotic alteration, which prevents or reduces undesirable side effects of known antidepressants.

In one aspect, the invention is aimed at the use of flumazenil for the manufacture of a medicament for the treatment of depressive disorders, particularly disorders including depressive symptoms, without psychotic alteration.

In another aspect, the invention is aimed at a method for the treatment of depressive disorders, particularly disorders including depressive symptoms, without psychotic alteration, comprising the administration of a therapeutically effective quantity of flumazenil to a subject who needs said treatment.

It has been found that flumazenil is effective in the treatment of depressive disorders, particularly of disorders including depressive symptoms, without psychotic alteration, causing a base improvement, especially at the level of GABA receptors, it shows no typical intrinsic effects (side effects or addictions) and creates neither a rebound effect nor dependency. It has further been found that significant results can be obtained with a very small quantity to be administered.

### Detailed Description of the Invention

The meaning of some terms and expressions within the context of the invention is provided below to aid in understanding this patent application.

The term "subject" relates to a member of a mammalian animal species, and includes but is not limited to domestic animals, primates and humans; preferably, the subject is a male or female human being of any age or race.

The term "treatment" as it is used herein relates to the reversal, inhibition of the progress or prevention of the disorder or condition to which it is applied, or of one or more symptoms of such disorder or condition.

The expression "depressive disorders" as it is used herein generally relates to any mental disorder presented with one or more depressive episodes, without psychotic alteration, and includes clinical disorders. In a particular embodiment, depressive disorders are related to disorders including depressive symptoms, without psychotic alteration, and includes those mental disorders admitted as such by the WHO, for example, the mental disorders included within groups F30-F48 in ICD10 [see, for example, "ICD10 - Mental and Behavioral Disorders- Clinical descriptions and guidelines for diagnosis", edited by MEDITOR (1992), hereinafter "ICD10"]. By way of illustration, said disorders including depressive symptoms, without psychotic alteration, include:
- bipolar disorder depressive episodes, such as current episode manic with psychotic symptoms, current episode mild or moderate depression (without somatic symptoms or with somatic symptoms), or current episode severe depression without psychotic symptoms, etc., such as those described and defined in "ICD10", pages 149-150, sections F31.2, F31.3 and F31.4, respectively;
- mild depressive episodes (without somatic symptoms or with somatic symptoms), moderate depressive episodes (without somatic symptoms or with somatic symptoms), severe depressive episodes (without somatic symptoms), other depressive episodes and unspecified depressive episodes, etc., such as those described and defined in "ICD10", pages 152-158, all of section F32 except F32.3;
- recurrent depressive disorders, current episode mild (without somatic symptoms or with somatic symptoms), current episode moderate (without somatic symptoms or with somatic symptoms), current episode severe (without somatic symptoms), recurrent depressive disorder currently in remission, other recurrent depressive disorders and unspecified recurrent depressive disorders, etc., such as those described and defined in "ICD10", pages 158-162, all of section F33 except F33.3;
- persistent mood (affective) disorders, such as cyclothymia, dysthymia, unspecified persistent mood (affective) disorders, etc., such as those described and defined in "ICD10", pages 162-165, all of section F34;
- recurrent brief depressive disorders, etc., such as those described and defined in "ICD10", pages 165-166, section F38.10; and
- depressive disorders included in "ICD10", sections F40 to F48, related to neurotic, stress-related and somatoform disorders, such as mixed anxiety and depressive disorder described in "ICD10", section F41.2; brief depressive reaction and prolonged depressive reaction as well as mixed anxiety and depressive reaction, described in sections F43.20, F43.21 and F43.22 respectively of "ICD10".

As has been previously mentioned, in a first aspect, the invention relates to the use of flumazenil in the manufacture of a medicament for the treatment of depressive disorders. In a particular embodiment, said depressive disorders comprise disorders including depressive symptoms, without psychotic alteration.

In a particular embodiment, flumazenil is administered sequentially at short time intervals in small quantities until a quantity that is therapeutically effective for the treatment of said depressive disorder, such as a disorder including depressive symptoms, without psychotic alteration, has been administered.

More specifically, the invention relates to the use of flumazenil for the manufacture of a medicament for the sequential administration at time intervals comprised between 1 second and 60 minutes, preferably between 30 seconds and 30 minutes, more preferably between 1 minute and 15 minutes, of quantities of flumazenil comprised between 0.01 and 2 mg, preferably between 0.05 and 1 mg, more preferably between 0.1 and 0.3 mg, until a therapeutically effective quantity of flumazenil, normally comprised between 0.5 mg and 4 mg, preferably between 1 and 3 mg/day, more preferably between 1.5 and 2.5 mg/day, for the treatment of the depressive disorder has been administered. In a particular embodiment, said depressive disorders comprise disorders including depressive symptoms, without psychotic alteration.

It must be taken into account that the suitable dose for each subject will be given by his or her age, weight, sex, physical condition and other medication that he or she may be taking, therefore the previously indicated values can be adjusted depending on these factors.

Flumazenil can be administered in combination with other medication used for the treatment of depressive disorders, particularly for the treatment of disorders including depressive symptoms, without psychotic alteration.

Although the daily therapeutically effective dose of flumazenil could be administered in a single administration, it has surprisingly been discovered that flumazenil can be safely and effectively administered to subjects with depressive disorders, for example, a subject with disorders including depressive symptoms, without psychotic alteration, in small quantities administered sequentially and separated by a relatively short time interval until a quantity of flumazenil that is therapeutically effective for the treatment of said depressive disorder, particularly said disorders including depressive symptoms, without psychotic alteration, has been reached. This discovery means that it is possible to administer flumazenil in successive small doses for the treatment of disorders including depressive symptoms, without psychotic alteration, in a very short time period, which reduces the risk of side effects in the subject and involves a better use of flumazenil for the treatment of typical symptoms of depressive disorders, for example, typical symptoms of disorders including depressive symptoms, without psychotic alteration. Other advantages of the use of flumazenil for the treatment of depressive disorders, and particularly of disorders including depressive symptoms, without psychotic alteration, consist of an absence of a rebound effect and dependency and of the base improvement at the level of GABA receptors on which it causes stable modifications, which mean a more prolonged effect of the drug.

In fact, the treatment with flumazenil of depressive disorders, particularly of disorders including depressive symptoms, without psychotic alteration, has at least the advantage that she improvement that it induces reaches a clinical level in less than one week. Likewise, the very low pharmacological iatrogenesis derived from its use is worth emphasizing, the duration thereof furthermore being very limited over time.

Example 1 shows that the administration to patients of 2 mg/day of flumazenil divided into doses of 0.2 mg every 3 minutes substantially improves the mood of the treated patients.

Therefore, in a particular embodiment, the invention relates to the use of flumazenil for the manufacture of a medicament for the sequential administration at 3-minute intervals of 0.2 mg of flumazenil until a therapeutically effective quantity of approximately 2 mg/day of flumazenil for the treatment of the depressive disorders has been administered. In a particular embodiment, said depressive disorders comprise disorders including depressive symptoms, without psychotic alteration.

Flumazenil can be administered by any suitable route of administration, for example, orally, nasally or parenterally, for which it will be formulated with the suitable excipients for the dosage form to be used. In a particular embodiment, flumazenil is administered intravenously (IV).

Flumazenil is very quickly metabolized when it is administered orally, much more quickly than when it is administered intravenously. Therefore, when flumazenil is administered orally, the dose must be adjusted such that a plasma concentration is reached similar to that obtained when a quantity of flumazenil greater than or equal to 0.2 mg is administered intravenously.

In another aspect, the invention relates to a method for the treatment of depressive disorders comprising the administration of a therapeutically effective quantity of flumazenil to a subject who needs said treatment, normally comprised between 0.5 and 4 mg/day, preferably between 1 and 3 mg/day, more preferably between 1.5 and 2.5 mg/day.

In a particular embodiment, said depressive disorders comprise disorders including depressive symptoms, without psychotic alteration.

In another particular embodiment, the method for the treatment of said depressive disorders, such as a disorder including depressive symptoms, without psychotic alteration, provided by this invention comprises the administration of a therapeutically effective quantity of flumazenil to a subject who needs said treatment, normally comprised between 0.5 and 4 mg/day, preferably between 1 and 3 mg/day, more preferably between 1.5 and 2.5 mg/day, distributed into quantities of flumazenil comprised between 0.01 and 2 mg, preferably between 0.05 and 1 mg, more preferably 0.1 and 0.3 mg and intended for their sequential administration at time intervals comprised between 1 second and 60 minutes, preferably between 30 seconds and 30 minutes, more preferably between 1 and 15 minutes, until said quantity of flumazenil that is therapeutically effective for the treatment of the depressive disorder has been reached. In a particular embodiment, said depressive disorders comprise disorders including depressive symptoms, without psychotic alteration.

In a specific embodiment, the invention provides a method for the treatment of depressive disorders, for example, a disorder including depressive symptoms, without psychotic alteration, comprising the administration to a subject who needs said treatment of approximately 2 mg/day of flumazenil, distributed into quantities of 0.2 mg of flumazenil intended for their sequential administration every 3 minutes until said quantity of approximately 2 mg/day of flumazenil has been reached.

The method for the treatment of depressive disorders, particularly of disorders including depressive symptoms, without psychotic alteration, provided by this invention is applicable to any subject who, when subjected to the treatment, does not have an acute or uncompensated disease, or is taking medication that is contraindicated with flumazenil. The method of treatment of depressive disorders, particularly of disorders including depressive symptoms, without psychotic alteration, provided by this invention generally starts with a complete medical and psychological examination. The condition of the subject is evaluated before and after the administration of flumazenil. In the event that the patient presents a panic attack, a suitable therapeutic agent, for example clomethiazole, can be administered before the administration of flumazenil. Flumazenil can be administered orally or IV, for example, by means of boluses containing the suitable quantity and observing the reaction in the patient. Once the treatment has ended, as part of the therapeutic program, the patient must follow a pharmacological treatment and, optionally hold meetings with his or her therapist to evaluate his or her evolution.

The following example illustrates the invention and must not be considered as limiting the scope thereof.

### EXAMPLE 1

### Treatment of patients with flumazenil at low doses and sequentially

### 1.1 Experimental Protocol

Three patients presenting a depressive disorder were subjected to treatment. Patients P01 and P02 reported associated cocaine consumption and the third patient (P03) reported associated alcohol consumption. The data relating to the SCL-90-R (Symptom Checklist 90 Revised, developed by Derogatis) scale is shown in Table 1:

**Table 1**

| Data relating to the SCL-90-R scale | | | | |
|---|---|---|---|---|
| Patient code | Age | Sex | SCL-90-R at the start | SCL-90-R at the end |
| P01 | 31 | Female | 78 | 0 |
| P02 | 35 | Male | 48 | 30 |
| P03 | 39 | Male | 92 | 25 |

The patients subjected to the treatment were previously provided with the suitable information and the corresponding informed consent was obtained from them.

During the assessment prior to the admission, a thorough psychopathological and medical evaluation was conducted first thing in the morning (9:00 AM). The medical examination included an electrocardiogram and a complete blood analysis, in which the different cell series (red blood cells, white blood cells and platelets) and the biochemical profile (glucose, creatinine, urea, cholesterol and the fractions thereof, triglycerides, alkaline phosphatase, GOT, GPT, GGT, LDH, total proteins, etc.) were studied. Criteria of exclusion from the treatment, such as pregnancy or uncompensated organic diseases were not found in any of the three cases.

The medication protocol of the three patients throughout their stay is shown in Table 2.

**Table 2**

| Protocol followed during the stay | | | |
|---|---|---|---|
| **Time** | **Day of admission** | **Day 2** | **Day of release** |
| 9:00 | | Clomethiazole 192 mg B1-B6-B12 Complex | Clomethiazole 192 mg B1-B6-B12 Complex |
| 11:00 | | Flumazenil 2 mg | Flumazenil 2 mg |
| 13:00 | Clomethiazole 192 mg B1-B6-B12 Complex | | |
| 16:30 | Flumazenil 2 mg | | |
| 19:30 | B1-B6-B12 Complex | B1-B6-B12 Complex | |
| 21:30 | Clomethiazole 384 mg | Clomethiazole 384 mg | |

The medication upon the release of the three patients was the following:
Vitamin B Complex: 1-1-0 (breakfast-lunch-dinner) for one month;
Clomethiazole: 192 mg for 15 days: 1-0-1 during the 1^{st} week, and 1-0-0 during the 2^{nd} week; and
Gabapentin: 300 mg, 1-1-1 for six months, according to assessment.

Flumazenil was administered in doses of 0.2 mg every 3 minutes (up to a total of 2 mg/day). This quantity per dose was established to reduce as much as possible undesirable side effects related to possible interactions with other drugs or psychopathologies. Before starting the first administration of flumazenil, a test consisting of the administration of a bolus of 0.1 mg of flumazenil was conducted to evaluate the reaction of the subject.

### 1.2 Results

### Results after the first administration of flumazenil

In one of the cases, patient P01 presented secondary symptoms associated to the administration of flumazenil, in the form of mild dizziness which disappeared without requiring any subsequent intervention, except a slight adjustment in the pace of application of the treatment.

The heart rate values of the patients did not undergo any significant increases during the administration of flumazenil, and the blood pressure values also remained within the range of normal, except for one of the patients (patient P03) who presented high blood pressure values from the start due to a prior hypertension disorder.

After this first administration, important changes were observed in the mood of the patients: the three patients reported a feeling of wellbeing and more calmness with respect to the time of the admission.

Changes in altered psychophysiological functions such as sleep also started: patient P01 reported "early waking" which did not occur on this first night, and patient P03, who reported difficulty in falling asleep, such sleep being intermittent and irregular, fell asleep early in the night.

### Results after the second administration of flumazenil

Secondary symptoms did not occur in this new administration of flumazenil, the feeling of dizziness experienced by patient P01 in the first application not occurring.

The cardiovascular values (heart rate and blood pressure) did not undergo significant changes either, the elevation of the blood pressure figures of patient P03 persisting.

Changes were observed in the mood of the three patients. In a general atmosphere of more calmness, relaxation and serenity, in the three cases the patients showed a better mood, greater capacity for attention and concentration, greater capacity for self-criticism and disease awareness, greater predisposition when making changes in their lives and establishing new projects for the future.

### Results after the third administration of flumazenil

As was expected, after this new administration of flumazenil there were no symptoms associated to such administration. Likewise, the cardiovascular values remained within normal parameters.

In the mood aspect, the changes already established after the second administration of flumazenil were confirmed, the improvement of attention, concentration and other superior faculties being more evident. The SCL-90-R scale was applied again, a reduction in the depression values being observed, as can be seen in Table 1.

The already mentioned existing sleep disorders were completely corrected.

## Claims

1. Use of flumazenil in the manufacture of a medicament for the treatment of depressive disorders.

2. The use according to claim 1, wherein said depressive disorders comprise disorders including depressive symptoms, without psychotic alteration.

3. The use according to claim 2, wherein said disorder including depressive symptoms, without psychotic alteration, is selected from:
- bipolar disorder depressive episodes, selected from current episode manic with psychotic symptoms, current episode mild or moderate depression (without somatic symptoms or with somatic symptoms), and current episode severe depression without psychotic symptoms;
- mild depressive episodes (without somatic symptoms or with somatic symptoms), moderate depressive episodes (without somatic symptoms or with somatic symptoms), severe depressive episodes (without somatic symptoms) and unspecified depressive episodes;
- recurrent depressive disorders, selected from current episode mild (without somatic symptoms or with somatic symptoms), current episode moderate (without somatic symptoms or with somatic symptoms), current episode severe (without somatic symptoms), recurrent depressive disorder currently in remission and unspecified recurrent depressive disorders;
- persistent mood (affective) disorders, selected from cyclothymia, dysthymia and unspecified persistent mood (affective) disorders;
- recurrent brief depressive disorders; and
- neurotic, stress-related and somatoform disorders, selected from mixed anxiety and depressive disorder, brief depressive reaction, prolonged depressive reaction and mixed anxiety and depressive reaction.

4. The use according to any of the previous claims, wherein said medicament is administered sequentially in doses at time intervals comprised between 1 second and 60 minutes, preferably between 30 seconds and 30 minutes and more preferably between 1 minute and 15 minutes.

5. The use according to claim 4, wherein the sequential intravenous administration of said medicament is carried out at intervals of approximately 3 minutes.

6. The use according to any of the previous claims, wherein said medicament is administered in quantities of flumazenil comprised between 0.01 and 2 mg per dose, preferably between 0.05 and 1 mg, more preferably between 0.1 and 0.3 mg until a quantity of flumazenil that is therapeutically effective for the treatment of said depressive disorder has been administered.

7. The use according to any of the previous claims, wherein the quantity of flumazenil that is therapeutically effective for the treatment of said depressive disorder or said disorder including depressive symptoms, without psychotic alteration, is comprised between 0.5 and 4 mg/day, preferably between 1 and 3 mg/day and more preferably between 1.5 and 2.5 mg/day of flumazenil.

8. The use according to claim 6, wherein said quantity of flumazenil that is therapeutically effective for the treatment of said depressive disorder or said disorder including depressive symptoms, without psychotic alteration, is approximately 2 mg/day.

9. The use according to any of the previous claims, wherein said medicament based on flumazenil is administered orally or parenterally, preferably intravenously.

10. Use of flumazenil in the manufacture of a medicament for the sequential intravenous administration of approximately 0.2 mg of flumazenil at time intervals of approximately 3 minutes, up to a quantity of 2 mg/day for the treatment of depressive disorders.

11. The use according to claim 10, wherein said depressive disorders comprise disorders including depressive symptoms, without psychotic alteration.

12. A method for the treatment of depressive disorders comprising the administration of a therapeutically effective quantity of flumazenil to a patient who needs said treatment.

13. The method according to claim 12, wherein said depressive disorders comprise disorders including depressive symptoms, without psychotic alteration.

14. The method according to claim 13, wherein said disorder including depressive symptoms, without psychotic alteration, is selected from:
- bipolar disorder depressive episodes, selected from current episode manic with psychotic symptoms, current episode mild or moderate depression (without somatic symptoms or with somatic symptoms), and current episode severe depression without psychotic symptoms;
- mild depressive episodes (without somatic symptoms or with somatic symptoms), moderate depressive episodes (without somatic symptoms or with somatic symptoms), severe depressive episodes (without somatic symptoms) and unspecified depressive episodes;
- recurrent depressive disorders, selected from current episode mild (without somatic symptoms or with somatic symptoms), current episode moderate (without somatic symptoms or with somatic symptoms), current episode severe (without somatic symptoms), recurrent depressive disorder currently in remission and unspecified recurrent depressive disorders;
- persistent mood (affective) disorders, selected from cyclothymia, dysthymia and unspecified persistent mood (affective) disorders;
- recurrent brief depressive disorders; and
- neurotic, stress-related and somatoform disorders, selected from mixed anxiety and depressive disorder, brief depressive reaction, prolonged depressive reaction and mixed anxiety and depressive reaction.

15. The method according to any of claims 12 to 14, wherein said medicament is administered sequentially in doses at time intervals comprised between 1 second and 60 minutes, preferably between 30 seconds and 30 minutes and more preferably between 1 minute and 15 minutes.

16. The method according to claim 15, wherein the sequential intravenous administration of said medicament is carried out at intervals of approximately 3 minutes.

17. The method according to any of claims 12 to 16, wherein said medicament is administered in quantities of flumazenil comprised between 0.01 and 2 mg per dose, preferably between 0.05 and 1 mg, more preferably between 0.1 and 0.3 mg until a quantity of flumazenil that is therapeutically effective for the treatment of said depressive disorder has been administered.

18. The method according to any of claims 12 to 17, wherein the quantity of flumazenil that is therapeutically effective for the treatment of said depressive disorder or said disorder including depressive symptoms, without psychotic alteration, is comprised between 0.5 and 4 mg/day, preferably between 1 and 3 mg/day and more preferably between 1.5 and 2.5 mg/day of flumazenil.

19. The method according to claim 17, wherein said quantity of flumazenil that is therapeutically effective for the treatment of said depressive disorder or said disorder including depressive symptoms, without psychotic alteration, is approximately 2 mg/day.

20. The method according to any of claims 12 to 19, wherein said medicament based on flumazenil is administered orally or parenterally, preferably intravenously.

21. A method for the treatment of depressive disorders comprising the sequential intravenous administration to a patient who needs treatment of approximately 0.2 mg of flumazenil at time intervals of approximately 3 minutes, up to a quantity of 2 mg/day.

22. The method according to claim 21, wherein said depressive disorders comprise disorders including depressive symptoms, without psychotic alteration.
